# EUROPEAN PATENT APPLICATION

(11) **EP 4 603 123 A1**
(43) Date of publication of application: **20.08.2025**
(21) Application number: 24305249.5
(22) Date of filing: 13.02.2024
(51) Int. Cl.: A61M 5/32

(54) **NEEDLE SHIELDS WITH UNIQUE IDENTIFYING INFORMATION**

(71) Applicant: Becton Dickinson France, 38800 Le Pont-de-Claix (FR)
(72) Inventor: PETIT, Louis, 38610 Venon (FR)
(74) Representative: Germain Maureau

(57) **Abstract**

A syringe assembly and a needle shield are disclosed herein. The needle shield includes an outer casing defining a cavity therein, an inner casing positioned at least partially within the cavity, with the inner casing configured to receive at least a portion of a needle therein. A label is positioned within the cavity and external to the inner casing, wherein the label includes at least one unique device identifier.

## Description

### FIELD OF THE INVENTION

The present disclosure relates generally to medical devices and systems and methods of use thereof for providing unique identifying information on such devices.

### BACKGROUND OF THE INVENTION

Traceability is an important aspect of medical devices for maintaining quality control, ensuring proper intended use, identifying or preventing tampering or damage, and other purposes throughout processes for manufacturing, assembly, delivery, and use for such devices. While existing traceability efforts may utilize identifying labels or tags on or about various surfaces of a medical device or its packaging, such exposed identifiers may be subject to damage or tampering. Additionally, the location of many existing identifiers on such devices may not be conducive to effective and efficient scanning/reading of the identifier.

The present disclosure provides systems, devices, and methods of use thereof for providing unique identifying information on such devices that are resistant to damage or tampering, and that are conveniently located and accessible for scanning or reading.

### SUMMARY OF THE INVENTION

In accordance with an embodiment of the present invention, a needle shield includes an outer casing defining a cavity therein, an inner casing positioned at least partially within the cavity, with the inner casing configured to receive at least a portion of a needle therein. A label is positioned within the cavity and external to the inner casing, wherein the label includes at least one unique device identifier.

In accordance with another embodiment of the present invention, the label is visible through the outer casing.

In accordance with another embodiment of the present invention, the at least one unique device identifier is visible through the outer casing.

In accordance with another embodiment of the present invention, the outer casing is at least partially transparent.

In accordance with another embodiment of the present invention, the at least one unique device identifier includes a machine readable code.

In accordance with another embodiment of the present invention, the machine readable code includes a data matrix having a substantially rectangular shape defining a longitudinal axis.

In accordance with another embodiment of the present invention, the outer casing defines a longitudinal axis substantially parallel to the longitudinal axis of the data matrix.

In accordance with another embodiment of the present invention, the at least one unique device identifier is printed on a surface of the label.

In accordance with another embodiment of the present invention, the at least one unique device identifier includes a plurality of unique device identifiers spaced on a surface of the label, wherein each of the plurality of unique device identifiers is the same as each of the other unique device identifiers of the plurality.

In accordance with another embodiment of the present invention, the label extends around a substantial portion of a perimeter of the inner casing.

In accordance with another embodiment of the present invention, the inner casing is constructed from an elastomeric material.

In accordance with another embodiment of the present invention, the outer casing is constructed from a material having a greater hardness than the elastomeric material of the inner casing.

In accordance with another embodiment of the present invention, the label is provided with an RFID tag.

In accordance with another embodiment of the present invention, a syringe assembly includes a syringe having a barrel and a needle, and a needle shield attached to the syringe. The needle shield includes an outer casing defining a cavity therein, an inner casing positioned at least partially within the cavity, with the inner casing configured to receive at least a portion of the needle therein, and a label positioned within the cavity, wherein the label includes at least one unique device identifier visible through the outer casing.

In accordance with another embodiment of the present invention, the unique device identifier includes at least one of a batch number, manufacturing line, test result, time stamp, expiry date, drug identification, process setting, or component batch number pertaining to a contents of the syringe.

In accordance with another embodiment of the present invention, the at least one unique device identifier includes a machine readable code having a substantially rectangular shape defining a longitudinal axis.

In accordance with another embodiment of the present invention, the barrel defines a longitudinal axis substantially parallel to the longitudinal axis of the machine readable code.

In accordance with another embodiment of the present invention, the at least one unique device identifier includes a plurality of identical unique device identifiers spaced apart from each other on the label.

In accordance with another embodiment of the present invention, the plurality of identical unique device identifiers includes a first row of unique device identifiers and a second row of unique device identifiers, and wherein the unique device identifiers of the second row are not radially aligned with the unique device identifiers of the first row.

In accordance with another embodiment of the present invention, a syringe assembly includes a syringe having a barrel and a needle, and a needle shield attached to the syringe. The needle shield includes an outer casing defining a sidewall and a cavity therein, an inner casing positioned at least partially within the cavity, with the inner casing configured to receive at least a portion of the needle therein, and a label positioned within the cavity between the sidewall of the outer casing and the inner casing. The label includes a plurality of unique device identifiers visible through the shield body, wherein the plurality of unique device identifiers includes a plurality of radially offset rows of unique device identifiers, and wherein the label extends around at least 75% of a perimeter of the inner casing.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings, wherein:
FIG. 1 is a side view of an example of a medical device constructed in accordance with the principles of the present disclosure;
FIG. 2 is cross-sectional partial assembly view of the medical device of FIG. 1;
FIG. 3 is a cross-sectional view of an example of a needle shield of the medical device of FIG. 1;
FIG. 4 is an illustration of an example of a label of the needle shield of FIG. 3; and
FIG. 5 is a partial assembly view of the needle shield of FIG. 3.

### DETAILED DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As used herein, the singular form of "a", "an", and "the" include plural referents unless the context clearly dictates otherwise.

Spatial or directional terms, such as "left", "right", "inner", "outer", "above", "below", and the like, relate to the embodiments or aspects as shown in the drawing figures and are not to be considered as limiting as the embodiments or aspects can assume various alternative orientations.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

All documents referred to herein are "incorporated by reference" in their entirety.

The term "at least" is synonymous with "greater than or equal to".

As used herein, "at least one of' is synonymous with "one or more of'. For example, the phrase "at least one of A, B, or C" means any one of A, B, or C, or any combination of any two or more of A, B, or C. For example, "at least one of A, B, and C" includes A alone; or B alone; or C alone; or A and B; or A and C; or B and C; or all of A, B, and C.

The word "comprising" and "comprises", and the like, does not exclude the presence of elements or steps other than those listed in any claim or the specification as a whole. In the present specification, "comprises" means "includes" and "comprising" means "including".

As used herein, the terms "parallel" or "substantially parallel" mean a relative angle as between two objects (if extended to theoretical intersection), such as elongated objects and including reference lines, that is from 0° to 5°, or from 0° to 3°, or from 0° to 2°, or from 0° to 1°, or from 0° to 0.5°, or from 0° to 0.25°, or from 0° to 0.1°, inclusive of the recited values.

As used herein, the terms "perpendicular", "transverse", "substantially perpendicular", or "substantially transverse" mean a relative angle as between two objects at their real or theoretical intersection is from 85° to 90°, or from 87° to 90°, or from 88° to 90°, or from 89° to 90°, or from 89.5° to 90°, or from 89.75° to 90°, or from 89.9° to 90°, inclusive of the recited values.

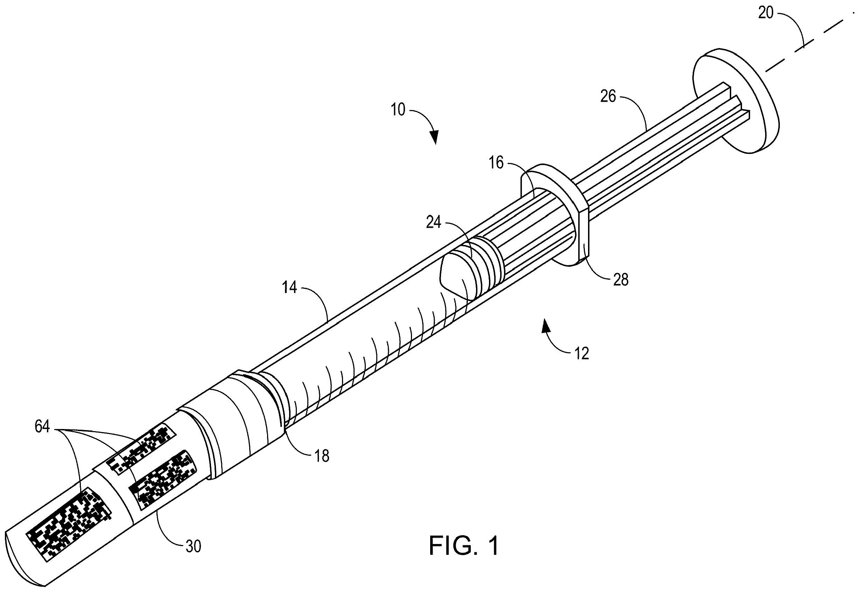

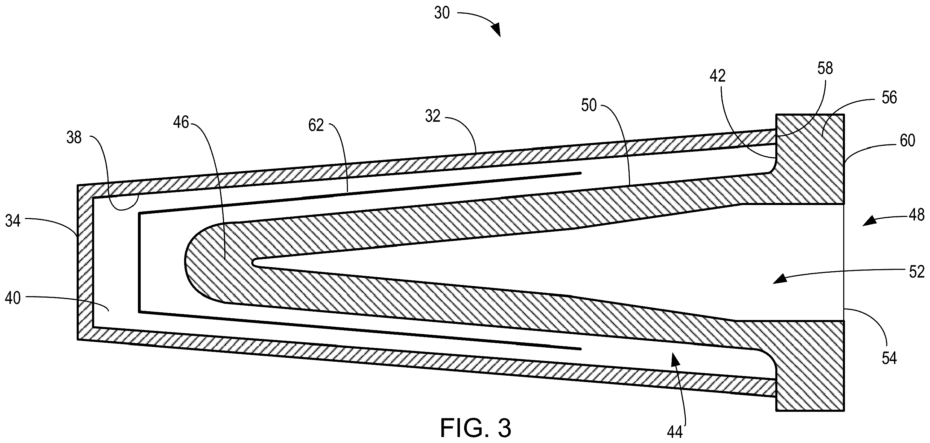

The present disclosure provides systems, devices, and methods of use thereof for providing unique identifying information on such devices that are resistant to damage or tampering, and that are conveniently located and accessible for scanning or reading. Referring now to the figures in which like reference designations refer to like elements, examples of a medical device 10 and aspects and components thereof are shown in FIGS. 1-5.

Referring now to FIGS. 1-3, the medical device 10 may generally include a syringe assembly 12. The syringe assembly 12 may include an elongate syringe barrel 14 having a proximal end 16, a distal end 18, and a reservoir therein configured to receive, store, or dispense one or more substances or fluids. The barrel 14 may define a longitudinal axis 20 extending along the length thereof. A needle 22 may extend from the distal end 18 and be in fluid communication with the reservoir of the barrel. A stopper 24 may be slidably positioned in fluid-tight engagement inside the reservoir of the syringe barrel 14, with stopper 24 connected to a plunger rod 26 to facilitate its operation. The stopper 24 is capable of moving fluid through the reservoir of the syringe barrel 14 upon movement toward the distal end 18, and is also capable of drawing fluid into the syringe barrel 14 upon movement of the stopper 24 away from the distal end 18 and towards the proximal end 16. The proximal end 16 of the barrel 14 may include a flange 28 so as to provide a structure and/or surface to apply a force to facilitate moving the plunger rod 26, and thus the stopper 24, with respect to the syringe barrel 14.

The medical device 10 may include a needle shield 30 attachable to the syringe assembly 12, where the needle shield 30 is configured or adapted to enclose or receive at least a portion of the needle 22 to protect or guard against inadvertent or undesired contact, piercing, or injection by the needle 22. The needle shield 30 may also prevent or reduce leakage of fluid contained within the syringe assembly 12. The needle shield 30 may be releasably attachable to the distal end 18 of the syringe barrel 14. The needle shield 30 may include or define an outer casing 32 having a closed first end 34, an open second end, defined by rim 56 and at least one sidewall 38 extending between the first end 34 and the second end defined by rim 56. The outer casing 32 may include or define an interior cavity 40 within or defined by the at least one sidewall 38, and an opening 42 at the open second end defined by rim 56 connected to or in fluid communication with the interior cavity 40.

The outer casing 32 may be constructed from one or more materials providing substantial rigidity and/or sufficient strength to resist potential damage during sterilization, storage, transport, and/or use of the medical device 10. Examples of such materials may include, e.g., a rigid plastic or polymeric material (e.g., polypropylene (PP), polyethylene (PE), polycarbonate (PC), polyethylene terephthalate (PET), polystyrene (PS), etc.). The needle shield 30 may have a substantially frustoconical or cylindrical shape, and/or may be otherwise sized and shaped to accommodate the desired portion of the needle 22 therein and to connect to the syringe assembly 12. The needle shield 30 may include or define a longitudinal axis parallel to the longitudinal axis 20 of the barrel 14 when the needle shield 30 is attached to the syringe assembly 12.

The needle shield 30 may include an inner casing 44 at least partially positionable within the cavity 40 of the outer casing 32 and configured or adapted to engage or receive a tip of the needle 22 when the needle shield 30 is fully seated on or coupled to the syringe assembly 12. The inner casing 44 may seal the tip of the needle 22 to prevent leakage of the contents of the barrel 14 of the syringe assembly 12. The inner casing 44 may include or define a closed first end 46, an open second end 48, and at least one sidewall 50 extending between the first and second ends 46, 48. The inner casing 44 may include or define an interior receptacle 52 within or defined by the at least one sidewall 50, and an opening 54 at the open second end 48 connected to or in fluid communication with the interior receptacle 52. The receptacle 54 may be sized and configured to receive at least a portion of the needle 22 of the syringe assembly 12. An internal diameter or dimension of the receptacle 52 may have a clearance fit relative to the needle 22, such that the receptacle 52 does not substantially abrade the needle 22 as the needle shield 30 is slid onto or otherwise coupled to the syringe assembly 12. The inner casing 44 may include or define a rim 56 extending from or located proximate to the second end 48. The rim 56 may have a larger width or diameter than the opening 54, and may circumscribe or surround the opening 54. The rim 56 may include or define a first surface 58 that seals or abuts a portion of the second end defined by rim 56 of the shield body 32. The rim 56 may include or define a second surface 60 that seals or abuts a portion of the syringe assembly 12 and/or barrel 14 when the needle shield 30 is attached to the syringe assembly 12. The sealing or positioning of the rim 56 or other portion of the needle shield 30 to the syringe assembly 12 and/or barrel 14 may serve as a sterile barrier to prevent contamination of the needle 22. The inner casing 44 may be formed of or constructed from any appropriate resilient, deformable, and/or elastomeric material such as, e.g., a rubber, thermoplastic elastomers (TPEs), bromobutyl rubber, etc.

The needle shield 30 may include a label 62 having one or more unique device identifiers 64 providing information correlated to or associated with an identifying characteristic of the medical device 10. For example, the one or more unique device identifiers 64 may present or incorporate unique, readable data regarding, e.g., batch number, manufacturing line, test results, time stamps, expiry date, drug identification, process settings, component batch number(s), etc. related to the medical device 10 and/or the contents thereof or therein. One or more readers or scanners can then be utilized to access the unique device identifiers 64 of each medical device 10, and a software and/or database solution may be provided to enable the storage and access of data linked to the unique device identifiers 64 of each label 62 and thus each medical device 10.

The label 62 may define, include, or be constructed from a segment of paper, film, or other medium adapted or configured to present visible information. The one or more unique device identifiers 64 may include one or more segments of machine readable code, such as, for example, bar codes, QR codes, data matrices, or other patterns or content conveying coded information. The one or more unique device identifiers 64 may be positioned on or otherwise incorporated with the label 62 by any applicable mechanism, including printing, etching, decal placement, overlay, or the like.

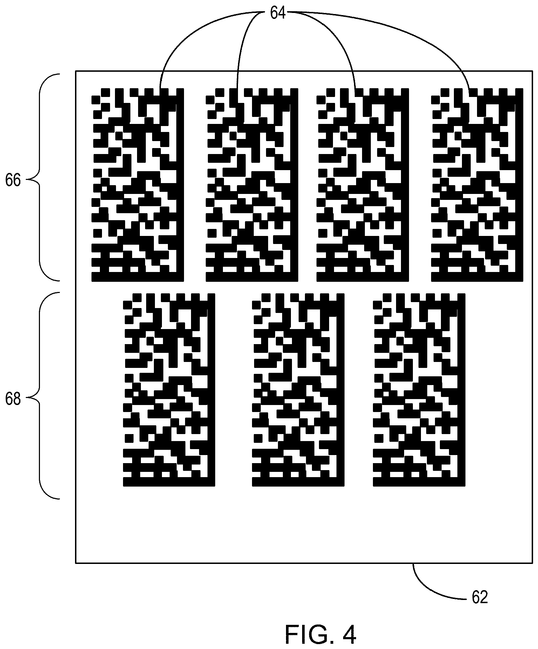

The label 62 may include a plurality of the unique device identifiers 64 to enable information associated with the unique device identifiers 64 to be scanned or read from multiple locations on and/or positions of the label. Each of the plurality of the unique device identifiers 64 may be identical or substantially similar to the other unique device identifiers 64 of the plurality such that the same information is conveyed or received from a scanning or reading of any individual unique device identifier 64 of the plurality. In the illustrated example of FIG. 4, a plurality of identical unique device identifiers 64 in the form of a QR code are spaced apart from each other across a surface of the label 62.

The positioning and/or spacing of the plurality of the unique device identifiers 64 may be configured to facilitate scanning or reading of at least one of the plurality of unique device identifiers 64 from a wide range of rotational and/or longitudinal positions of the label 62. For example, the plurality of identical unique device identifiers 64 may constitute or include a plurality of rows of unique device identifiers 64 that are longitudinally and/or laterally offset from each other. Continuing to refer to FIG. 4, the plurality of identical unique device identifiers 64 may include a first row 66 of unique device identifiers 64 and a second row 68 of unique device identifiers 64. Each of the unique device identifiers 64 in the second row 68 is offset and/or not aligned with the unique device identifiers 64 of the first row 66, thereby increasing the readability of at least one of the unique device identifiers 64 in either the first row 66 or second row 68 irrespective of the specific rotational and/or longitudinal position of the label 62 and the needle shield 30. The quantity and/or configuration of the rows of the unique device identifiers 64 may vary depending on the applicable dimensions or sizing of the components of the medical device 10 for a particular application.

The unique device identifier 64, whether presented in a single or plurality configuration, may be shaped to facilitate scanning or reading. For example, the unique device identifier 64 may have a substantially rectangular shape having a longitudinal axis or length substantially aligned with or substantially parallel to the longitudinal axis 20 of the barrel 14 and/or the needle shield 30, as described herein. In such a non-limiting example, the longitudinal alignment and substantially rectangular shape of the unique device identifier 64 reduces the likelihood that a radial curvature or circumference of the needle shield 30 interferes with or reduces the ability to scan or read the unique device identifier 64 on the label 62 of the needle shield 30.

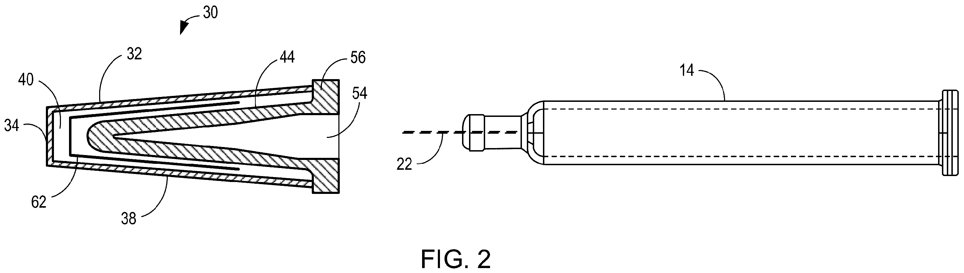

Now referring to FIGS. 2-3, the label 62 may be positioned within the interior cavity 40 of the outer casing 32 such that at least a portion of the label 62 is visible through the outer casing 32. At least a portion of the outer casing 32 may be transparent or sufficiently optically transmissible such that the unique device identifier 64 of the label 62 can be scanned or read from outside of or exterior to the outer casing 32. The label 62 may be disposed between an inner surface of the sidewall 38 of the outer casing 32 and an exterior surface of the sidewall 50 of the inner casing 44. In this configuration, the label 62 is substantially protected within the needle shield 30 in order to avoid damage, wear, tampering, etc., while still providing effective readability by an appropriate scanner or reader.

The label 62 may be sized, shaped, and/or otherwise configured to traverse or extend around a substantial portion of the perimeter or circumference of the needle shield 30, which improves scanning or reading the label from a wide range of positions or orientations of the needle shield 30 and thus, the medical device 10. In one example, the label 62 may extend along or wrap around at least 50% or 180° of the perimeter or circumference of the inner casing 44 within the outer casing 32. In another example, the label 62 may extend along or wrap around at least 75% or 270° of the perimeter or circumference of the inner casing 44 within the outer casing 32. In another example, the label 62 may extend along or wrap around substantially the entire perimeter or circumference of the inner casing 44 within the outer casing 32.

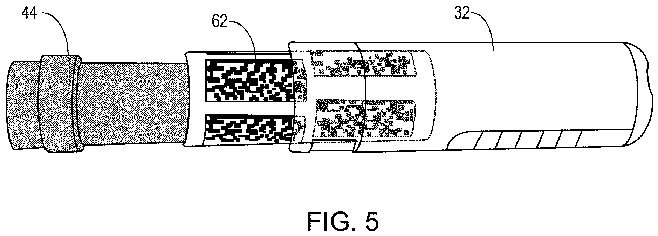

The assembly or positioning of the label 62 within the needle shield 30 may be achieved in various ways. In one non-limiting example shown in FIG. 5, the label 62 may be positioned within the cavity 40 of the outer casing 32, with the inner casing 44 subsequently inserted into the cavity 40 and sealed to the outer casing 32 to enclose or encase the label 62 therein. In another example, the label 62 may be adhered to the sidewall 38 of the outer casing 32 prior to insertion of the inner casing 44 into the outer casing 32. In another example, the label 62 may be adhered to the sidewall 50 of the inner casing 44, with the inner casing 44 subsequently inserted and sealed to the outer casing 32. Combinations and variations of such assembly procedures are contemplated to provide the configurations and benefits disclosed herein.

In one example, the unique identifier 64 may be used in conjunction with an RFID tag (not shown). Such an implementation allows for no line-of-sight reading and mass reading of information related to the medical device 10 and/or the contents thereof or therein utilizing the RFID tag along with the prevention of cross-reading with a basic reading system utilizing the label 62 including the unique identifier 64. In such an example, the RFID tag may be positioned between the sidewall 50 of the inner casing 44 and the sidewall 38 of the outer casing 32. For example, the RFID tag may adhere to the sidewall 50 of the inner casing 44 by adhesive bonding, or the RFID tag may adhere to the sidewall 38 of the outer casing 32 by adhesive bonding. The RFID tag is in a form of wet inlay, dry inlay, or pressure sensitive label.

RFID Wet Inlays and RFID Dry Inlays can comprise a substrate, for example made of paper or Polyethylene terephthalate (PET). The RFID tag can be disposed on one side of the substrate. RFID Wet Inlays and RFID Dry Inlays can further comprise at least one protective layer on top of the RFID tag. The protective layer can be a siliconized paper. In such an example, the label 62 comprising the unique identifier 64 is positioned on the protective layer. Alternatively, the unique identifier 64 is provided directly on the protective layer.

RFID Wet Inlays are described as "wet" as they include an adhesive layer on the other side of the substrate and a backing paper, for example with a silicon liner. RFID Dry Inlays are described as "Dry" due to their lack of adhesive backing. The RFID tag may be a Low Frequency Radio Frequency Identification (LF-RFID) tag. Low frequencies are usually about 30 KHz to 300 KHz. An RFID reader can, for example, read the LF-RFID tag at a distance up to about ten cm. alternatively, the RFID tag is a High Frequency Radio Frequency Identification (HF-RFID) tag. High frequencies are usually about 1-15 MHz. An RFID reader can, for example, read the HF-RFID tag at a distance about one meter. Alternatively, RFID tag may be a High-Frequency Near Field Communication (HF-NFC) tag. An NFC reader can, for example read, the HF-NFC tag at a distance up to a few centimeters. HF-NFC differs from HF-RFID in that it can be read by a NFC smartphone. Still further, the RFID tag may be an Ultra High Frequency Radio Frequency Identification (UHF-RFID) tag. Ultra-high frequencies are usually about 400-1000MHz. An RFID reader can for example read the UHF-RFID tag at a distance about fifteen meters.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A needle shield, comprising:
an outer casing defining a cavity therein;
an inner casing positioned at least partially within the cavity, the inner casing configured to receive at least a portion of a needle therein; and
a label positioned within the cavity and external to the inner casing, wherein the label includes at least one unique device identifier.

2. The needle shield of claim 1, wherein the label is visible through the outer casing, and optionally the at least one unique device identifier is visible through the outer casing, and further optionally the outer casing is at least partially transparent.

3. The needle shield of any of claims 1-2, wherein the at least one unique device identifier includes a machine readable code, and optionally the machine readable code includes a data matrix having a substantially rectangular shape defining a longitudinal axis.

4. The needle shield of any of claims 1-3, wherein the outer casing defines a longitudinal axis substantially parallel to the longitudinal axis of the data matrix.

5. The needle shield of any of claims 1-4, wherein the at least one unique device identifier is printed on a surface of the label.

6. The needle shield of any of claims 1-5, wherein the at least one unique device identifier includes a plurality of unique device identifiers spaced on a surface of the label, wherein each of the plurality of unique device identifiers is the same as each of the other unique device identifiers of the plurality.

7. The needle shield of any of claims 1-6, wherein the label extends around a substantial portion of a perimeter of the inner casing, and optionally wherein the inner casing is constructed from an elastomeric material.

8. The needle shield of any of claims 1-7, wherein the outer casing is constructed from a material having a greater hardness than the elastomeric material of the inner casing.

9. The needle shield of any of claims 1-8, wherein the label is provided with an RFID tag.

10. A syringe assembly, comprising:
a syringe having a barrel and a needle; and
a needle shield attached to the syringe, the needle shield comprising:
an outer casing defining a cavity therein;
an inner casing positioned at least partially within the cavity, the inner casing configured to receive at least a portion of the needle therein; and
a label positioned within the cavity, wherein the label includes at least one unique device identifier visible through the outer casing.

11. The syringe assembly of claim 10, wherein the unique device identifier includes at least one of a batch number, manufacturing line, test result, time stamp, expiry date, drug identification, process setting, or component batch number pertaining to a contents of the syringe.

12. The syringe assembly of any of claims 10-11, wherein the at least one unique device identifier includes a machine readable code having a substantially rectangular shape defining a longitudinal axis, and optionally wherein the barrel defines a longitudinal axis substantially parallel to the longitudinal axis of the machine readable code.

13. The syringe assembly of any of claims 10-12, wherein the at least one unique device identifier includes a plurality of identical unique device identifiers spaced apart from each other on the label.

14. The syringe assembly of any of claims 10-13, wherein the plurality of identical unique device identifiers includes a first row of unique device identifiers and a second row of unique device identifiers, and wherein the unique device identifiers of the second row are not radially aligned with the unique device identifiers of the first row.

15. A syringe assembly, comprising:
a syringe having a barrel and a needle; and
a needle shield attached to the syringe, the needle shield comprising:
an outer casing defining a sidewall and a cavity therein;
an inner casing positioned at least partially within the cavity, the inner casing configured to receive at least a portion of the needle therein; and
a label positioned within the cavity between the sidewall of the outer casing and the inner casing, wherein the label includes a plurality of unique device identifiers visible through the shield body, wherein the plurality of unique device identifiers includes a plurality of radially offset rows of unique device identifiers, and wherein the label extends around at least 75% of a perimeter of the inner casing.
